# EUROPEAN PATENT APPLICATION

(11) **EP 1 526 139 A1**
(43) Date of publication of application: **27.04.2005**
(21) Application number: 03024329.9
(22) Date of filing: 24.10.2003
(51) Int. Cl.: C07J 5/00, C07J 3/00, C07J 31/00, C07J 75/00

(54) **A process for preparing highly pure androstane 17-beta-carboxylic acids and androstane 17-beta-carbothioic acid fluoromethyl esters**

(71) Applicant: S.N.I.F.F. Italia S.P.A., 06073 Corciano (IT)
(72) Inventor: Vetturini, Emanuela, 06100 Castel del Piano (IT); Farnesi, Sara, 06055 Marsciano (IT)
(74) Representative: Gislon, Gabriele

(57) **Abstract**

The present invention relates to an oxidation process for preparing the androstane 17-β-carboxylic acid of general formula (I) with a high purity degree by oxidative demolition of the carbon atom 21 of the compound of general formula (II) by using hydrogen peroxide in a basic environment in a polar solvent optionally in the presence of water.

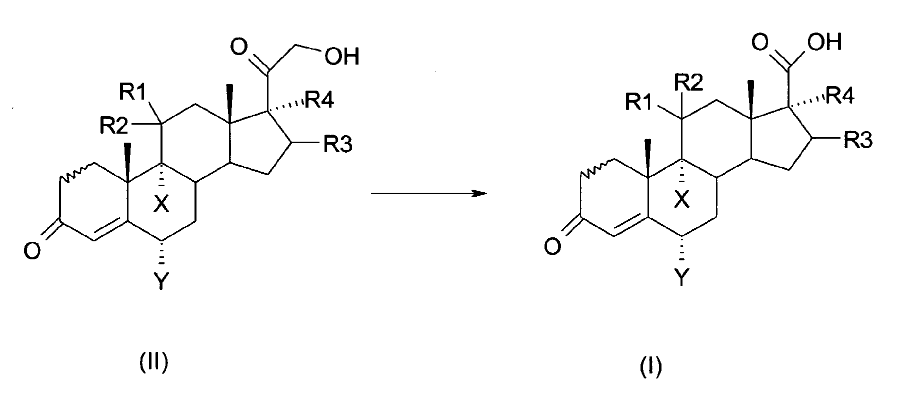

## Description

### FIELD OF THE INVENTION

The present invention relates to a process for preparing highly pure androstane 17-ß- carboxylic acid, useful in the synthesis of 17-ß-carbothioic acid fluoromethyl esters of androstane and in particular of fluticasone propionate.

### BACKGROUND OF THE INVENTION

Fluticasone propionate pertains to the class of androstane 17-ß-caqrbothioic esters having antiinflammatory activity.

US 4,335,121 GB 2,088,877, WO0116722, WO02/08243 disclose syntheses of fluticasone propionate from commercial grade flumethasone, namely (6α,11β,16α)-6,9-difluoro-11,17,21 -trihydroxy-16α-methyl-pregna-1,4-diene-3,20- 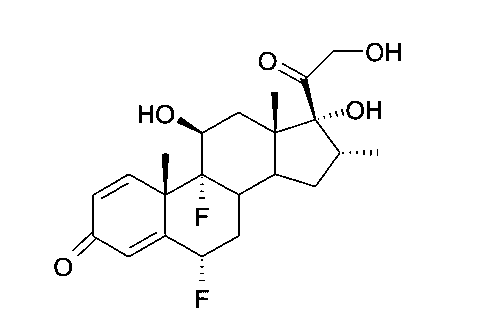 dione). These syntheses essentially comprised the following steps:
1) oxidation of the carbon atom in position 21 of flumethasone of formula to obtain (6α,11β,16α)-6,9-difluoro-11,17,dihydroxy-16α-methyl-androst-1,4-dien 3-one 17-β-carboxylic acid, 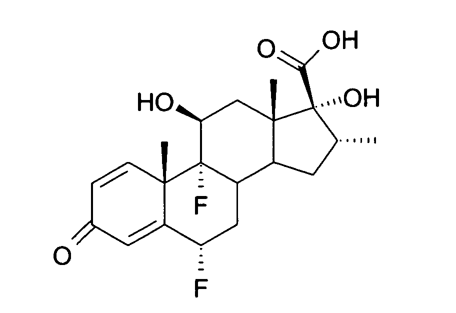
2) Acylation of the 17-α-hydroxy group with propionyl halide or anhydride thereby obtaining the corresponding 17-α-propionyl ester, 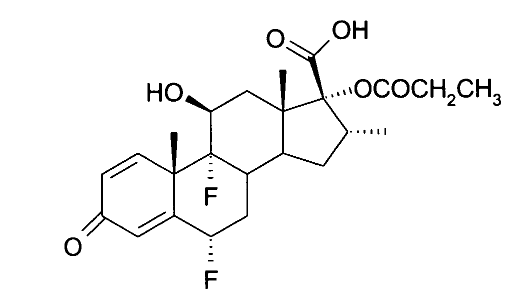
3) Conversion of the 17-α-propionyl ester 17-β-carboxylic acid to the corresponding 17-α-propionyl -17-β-carbothioic acid, 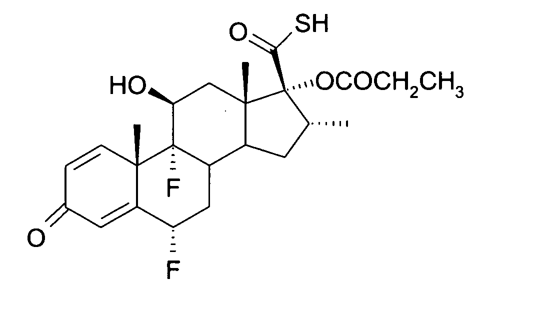
4) esterification of the 17-β-carbothioic acid thereby obtaining fluticasone propionate,

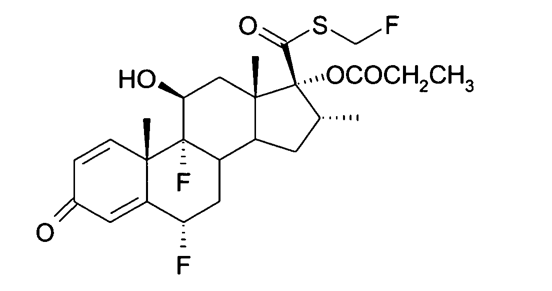

Alternatively as disclosed in WO02/08243 the acylation reaction of the 17-α-hydroxy group is carried out on the 17-α-hydroxy-17-β-carbothioic acids and before the final esterification (step 4) of the carbothioic acid.

In the aforementioned prior art documents the oxidation of the carbon atom in position 21 (step 1) is carried out by using periodic acid and the esterification of the carbothioic acid (step 4) is achieved by using clorofluoromethane.

In any case these syntheses suffer from a series of drawbacks.

First of all these syntheses encompass the use of commercial grade flumethasone which contain relevant amounts of impurities, which must be removed by using chromatography namely a type of purification not realisable on an industrial scale, crystallisation processes or even as described in WO01/62722 by carrying out a dehalogenation reaction with palladium catalysts, namely treatments bringing to an unavoidable yield decrease.

The aforementioned purification processes are necessary otherwise after the oxidation reaction realised with periodic acid it is impossible to obtain a 17-β-carboxylic acid with an appreciable purity degre.

In addition the use of the extremely reactive chlorofluoromethane in the final esterification step to obtain fluticasone propionate brings to the formation of an impure product, which must be subjected to a series of crystallisation treatments to the detriment of reaction yields.

The need was felt to overcome the aforementioned drawbacks in particular of the fluticasone propionate synthesis.

### SUMMARY OF THE INVENTION

The Applicant has now unexpectedly found that the aforementioned drawback can be easily overcome, by carrying out the oxidation reaction of the 21 carbon in the aforementioned step 1 using as an oxidising agent hydrogen peroxide.

Therefore object of the present invention is a process for preparing a 17-β-carboxylic acid of general formula (I) 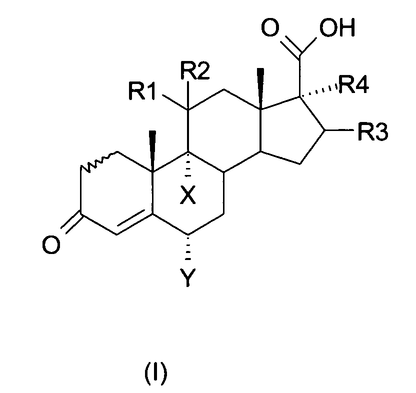 Wherein the symbol: 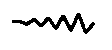

indicates the presence of a single or a double bond, R1 and R2 respectively represent a hydroxy group and a hydrogen atom or they form together an oxo group, X and Y equal or different from each other are Cl, F, R3 respectively represents the 16α or 16β methyl, R4 is a hydroxy or a C1-C6 alkanoyloxy group, comprising the oxidation reaction at the 21 carbon atom of the compound of general formula (II) 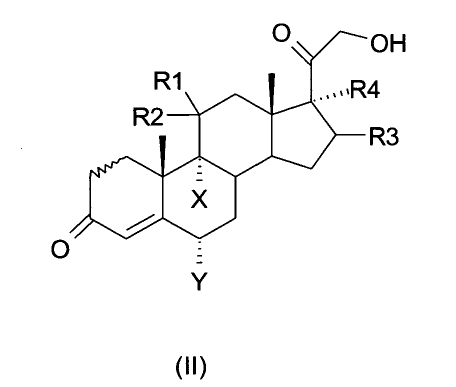 wherein R1, R2, X, Y, R3 and R4 and the symbol 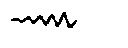

have the aforementioned meanings, characterised in that said oxidation is carried out by using hydrogen peroxide as the oxidising agent

In fact by carrying out the oxidation reaction according to the present invention utilising in particular technical grade flumethasone(titre ranging from 85-95%) it is possible to obtain the corresponding 17β-carboxylic acid in a decidedly much higher purity degree than that obtained by carrying out the oxidation reaction with periodic acid on the same starting material (see comparative example 3A).

The Applicant has also unexpectedly found that by using in the synthesis of fluticasone propionate in the final esterification step of the corresponding 17β-carbothioic acid bromofluoromethane in place of chlorofluoromethane it is possible to obtain a raw fluticasone propionate having a purity degree of 99.2%, wherein the content of the single impurities present is not higher than 0.3%.

Therefore the present invention relates to a process for preparing fluticasone propionate comprising reacting 17-β-carbothioic acid of formula (VI) 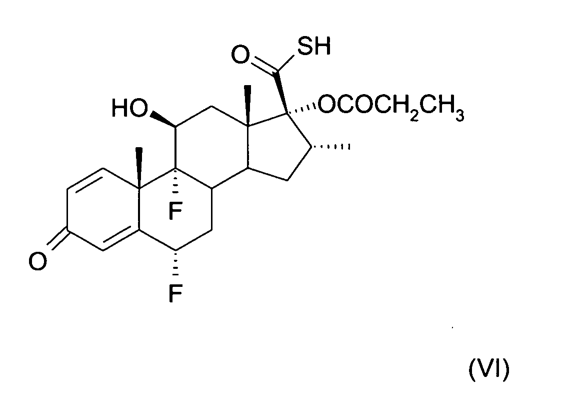 with bromofluoromethane

### DETAILED DESCRIPTION OF THE INVENTION

The oxidation reaction of the 21 carbon atom of the compound of general formula (II) is preferably carried out in the presence of a base in a polar solvent, optionally in admixture with water.

The molar ratio of hydrogen peroxide/compound (II) is preferably comprised between 5:1 and 50:1, more preferably between 10:1 and 20:1. 35% hydrogen peroxide is preferably used in the process according to the present invention. The base is preferably selected from the class consisting of: alkaline metal carbonate or alkaline hydrogen carbonate salts, such as lithium carbonate lithium hydrogen carbonate, sodium carbonate, sodium hydrogen carbonate, potassium carbonate, potassium hydrogen carbonate, cesium carbonate, alkaline metal phosphate or alkaline metal hydrogen phosphate salts, mono and dialkylamines such as diisopropylamine, trialkylamine such as triethylamine, heterocyclic amines as for example imidazol, pyridine, pyridazine, pyrimidine, pyrazine, bicyclic amines such as DBN (diazabicyclo[5.4.0]undec-7-ene) DBN (diazabicyclo[4.3.0]non-5-ene).

The polar solvent is preferably selected from an aprotic dipolar solvent such as dimethylacetamide dimethyl formamide, protic polar solvents such as C1-C4 alcohols, aprotic polar solvent such as tetrahydrofuran and dioxane, and mixtures thereof or possibly mixtures of said solvent with water. The temperature is not a critical parameter in the oxidation process according to the present invention.

In fact although the oxidation reaction according to the present invention can be easily carried out at room temperature, the same process can be also carried out at lower or higher temperatures than room temperature. The reaction time is a function of the specific starting compound of formula (II) and also of the temperature and is generally comprised between 1 and 18 hours.

According to a particularly preferred embodiment when flumethasone is used as the compound of formula (II), the oxidation process is carried out in the presence of potassium carbonate in a mixture of dimethylformamide and methanol at a temperature of 10°C.

The oxidation process according to the present invention can be used in the preparation of important steroids having antiinflammatory activity such as fluticasone and esters thereof and in particular fluticasone propionate.

According to a particularly preferred embodiment the process according to the present invention is used for the preparation of the intermediate of formula (I) wherein X=Y=F, R1=β-OH, R2=α-H, R4 =OH or propionyloxy, R3 = α-methyl and the symbol: 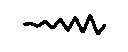

indicates a double bond,
which is used in the preparation of fluticasone propionate.

In this case the process according to the present invention may also contemplate the acylation reaction of the 17-α-hydroxy function to obtain the 17- α - propionyloxy ester.

Said acylation of the 17 hydroxy function in the process according to the present invention, can be carried out either on flumethasone, before or after said oxidation reaction.

Said acylation can be carried out by using conventional acylation reactants such as propionyl anhydride or propionyl halides.

The acylation step of the 17-α-hydroxy group, may also be carried out after the conversion of the 17-β-carboxylic acid to 17-β-carbothioic acid.

According to a particularly preferred embodiment of the process according to the present invention the acylation reaction is carried out on flumethasone before the oxidation reaction and it encompasses the following steps:
a) reacting flumethasone, namely the compound of formula (II) wherein X=Y=F, R1=β-OH, R2= α-H, R4 =OH R3 = α-methyl and the symbol:
   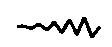 indicates a double bond,
   with a trialkyl orthopropionate of formula (III): 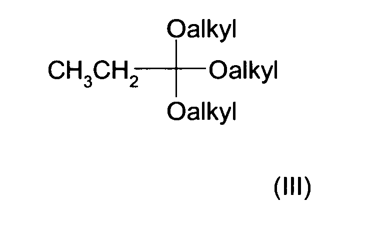 wherein "alkyl"means a C1-C5 linear or branched alkyl residue, preferably methyl, ethyl, propyl, butyl, even more preferably ethyl in an aprotic polar solvent preferably tetrahydrofuran, dioxane, ethyl acetate, more preferably ethyl acetate, preferably at a temperature ranging from 15°C to the reflux temperature and more preferably at room temperature, thereby obtaining the compound of formula (IV) wherein "alkyl" has the aforementioned meanings: 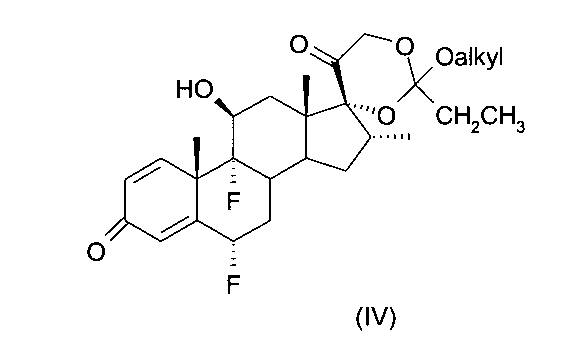
b) hydrolysing the cyclic adduct of formula (IV), in the presence of an organic carboxylic acid preferably acetic acid, in an alcoholic solvent preferably methanol, ethanol, n-propanol, isopropanol, butanol, or mixture thereof optionally in the presence of an apolar solvent preferably dichloromethane or chloroform thereby obtaining the compound of formula (V) 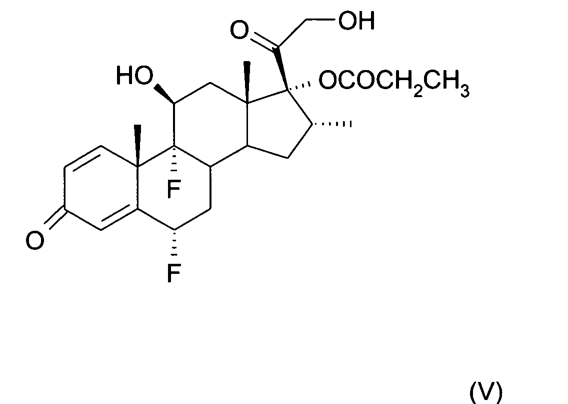 in other words the compound of formula (II) having X=Y=F, R1=β-OH, R2=α-H, R3=α-methyl, R4=OCOCH2CH3 and the symbol 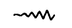

   indicates a double bond.

Preferably said hydrolysis is carried out in methanol at a temperature comprised between 20 and 60°C, even more preferably at 42°C.

The process, further subject of the present invention for preparing fluticasone propionate comprising the reaction with carbothioic acid of formula (VI) with bromofluoromethane is preferably carried out by adding at portions a diprotic polar solvent preferably a C2-C5 alkylamide of a carboxylic acid more preferably dimethyl acetamide and dimethyl formamide, in which bromofluoromethane was previously dissolved to a suspension of (VI) in the same solvent. The reaction temperature is preferably comprised between -20 and +30°C and the reaction time depends on the temperature and is comprised between 1 and 12 hours.

The carbothioic acid of formula (VI) may be prepared from the corresponding 17β-carboxylic acid according to the following reaction scheme: 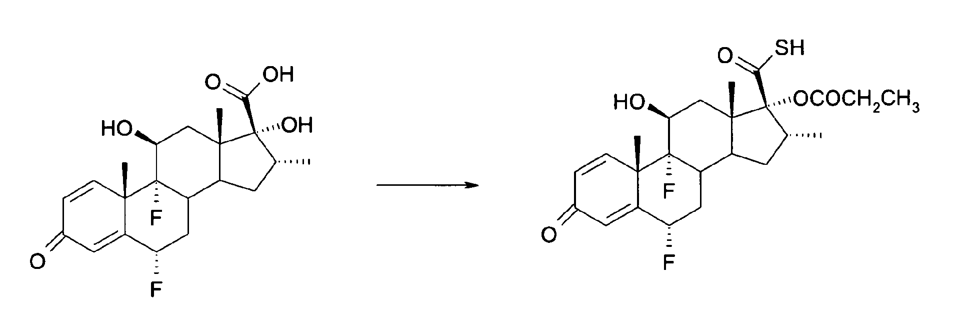 encompassing a treatment of the 17β-carboxylic acid with hydrogen sulfide gas in the presence of a coupling agent such as carbonyl imidazole as described in WO02/08243, or alternatively by reacting the aforementioned 17β-carboxylic acid with thiocarbamoyl chloride of formula (VII): 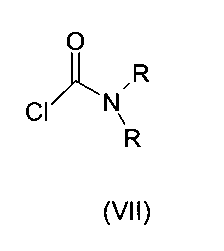 wherein R is a C1-C6 alkyl or alkenyl radical as described in US 4,335,121 or WO01/62722 and subsequently hydrolising in an acid environment as disclosed in US 4,335,121, or in a highly basic environment as described in WO01/62722 according to the following reaction scheme:

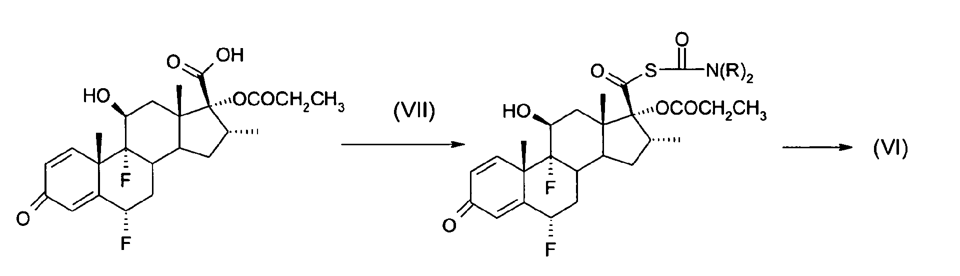

The crude fluticasone propionate prepared according to the process of the present invention, may be subjected to at least one crystallisation in order to increase it purity degree.

Preferably it is subjected to two crystallisations.

According to a particularly preferred embodiment the first crystallisation is conducted with a process comprising:
i) dissolving the raw product under under reflux in tetrahydrofurane/di-chloromethane in volume ratio 5:1,
ii) evaporating dichloromethane, favouring the precipitation of the purified product,
iii) filtering and drying the purified product.

The second crystallisation step comprises the following steps:
i) dissolving the raw product under reflux in ethylacetate/butanol in volume ratio 5:11
ii) cooling the solution obtained to precipitate the purified product,
iii) filtering and drying the precipitated product.

These treatments permit to obtain a final product with a purity degree higher than 99.5% preferably of 99.7%, wherein each single impurity is contained in an amount not exceeding 0.07%.

The following example of the process according to the present invention are herewith reported for illustrative but not limitative purposes.

### EXAMPLE 1

### 6α,9α,Difluoro-11β-dihydroxy-16α-methyl-pregna-17, 21-ethylorthopropionate-1,4diene-3,20-dione(IV)

Triethylorthopropionate(4.16ml, 20.7 mmol) and paratoluensulfonic acid (64.0mg), were added to a suspension of technical grade flumethasone (titre in flumethasone 95%) (6.4g, 15.6mmol) and the mixture obtained was left to react. Once the reaction was completed, ethyl acetate was partially removed by evaporation under reduced pressure. The concentrated reaction mixture was dissolved in methanol (200 ml) and then precipitated in demineralised water (600 ml) previously cooled to 0°C. After 2 hours the suspension was filtered an the precipitate thus recovered (5.0g 65%yield), was dried in an oven at 60°C. This product was used directly without undergoing further purification treatment.

Only a small portion of said product was crystallised from dichloromethane/methanol for carrying out on said sample IR analysis whose results are reported herein below:
IR (KBr, cm⁻¹): 3340,1723, 1665,1620, 1608, 1126
¹H-NMR(CDCl₃, 200 MHz), ppm: 3.97(2H), 3.47(2H), 1.75(2H), 1.17 (3H),0.92 (3H)
¹³C-NMR(CDCl₃,200 MHz) ppm: 203.64, 112.20, 168.23, 48.05, 158.23, 14.73, 7.66.

### EXAMPLE 2

### 6α,9α,Difluoro-11β,17α,21-trihydroxy-16α-methyl-17,propionate-pregn-1,4-diene-3,20-dione(V)

1.85 g acetic acid dissolved under magnetic stirring in 7.5 ml of bidistilled water were added to a suspension of (IV) (7.4 g, 14.1 mmol) in methanol (1800 ml) maintained at 42°C under Nitrogen flow. Once the reaction is completed half volume of methanol was distilled and the product was precipitated by adding bidistilled water (1000 ml) under vigorous stirring and then cooling the suspension obtained to 5°C.The product (V) obtained after filtration (5.75g, 87%) was dried in an oven at 60°C and used as such in the successive step.

Only a small portion of said product was crystallised from dichloromethane/methanol for carrying out on said sample IR analysis whose results are reported herein below:
¹³C-NMR(CDCl₃,200 MHz) ppm170.19(C=O) propionile; 27.00 (C(O)-CH₂CH₃); 9.18 (C(O)-CH₂CH₃)

### EXAMPLE 3

### 6α,9α,Difluoro-11β-dihydroxy-16α-methyl-3-oxo-17α(propionyloxy)androsta-1,4-diene-17β-carboxylic acid (I) wherein R1=β-OH, R3=α-methyl, X=Y=F, R4 =OCOCH2CH3 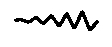

*indicates a double bond*

A first portion (5.3 ml) of (35%) hydrogen peroxide was added at 0°C to a suspension of (V) (7g, 17.0 mmol) and potassium carbonate (13.3g, 125.5 mmol) in dimethylformamide (210 ml). Then methanol (15.5 ml) was added and subsequently the second portion of hydrogen peroxide (3.5ml). The reaction mixture was left to react at 10°C until complete conversion of the starting product and subsequently precipitated in water (600 ml). The resulting salt coming from the reaction was washed with ethylacetate (2x400 ml). Afterwards (36%)hydrochloric acid was added until precipitation of the reaction product (pH≈1). The suspension was cooled to 0-5°C for 2 hours, filtered washed with water (2x300 ml) and dried in an oven at 60°C.The reaction yield is 78% and the purity of 99.5%.

Only a small portion of said product crystallised from dichloromethane/methanol was utilised for the physical chemical characterisation.
¹H-NMR(CDCl₃, 200MHz) ppm: 0.88(d,3H); 1.02 (s,3H);1.50 (s, 3H); 5.63(m,1H);6.10(s,1H); 6.30 (d, 1 H); 7.25 (d,2H).

### COMPARATIVE EXAMPLE 3A

5.0 g of 6α,9α-difluoro-11-β,17α,21-trihydroxy-17-propionate- pregn-1,4 diene-3,20 dione (titre in flumethasone 95%) (12.2mmol) was suspended in 50 ml tetrahydrofurane and stirred at 25°C. Then a periodic acid solution prepared by dissolving 12.5g (54.8 mmol) of said acid in 30 ml of water was dropped in 1 hour. The mixture was left under stirring for 3 hours and thereafter precipitated by addition of 500 ml water. The mixture thus obtained was cooled to a temperature of 0-5°C for at least 2 hours under stirring.

The mixture was then filtered and the precipitated obtained was washed with water (300 ml). The obtained products was dried in an oven at 50°C. The reaction yield was 87.6% and the final product ha a purity degree of 93.5%.

4 g of the raw product was purified by dissolution thereof in 106.6 ml of tetrahydrofurane under reflux, re-concentration of the solution obtained up to 40 ml volume and addition by means of a dripping funnel in about 1 hour of 25 ml bidistilled water.

The obtained suspension was then cooled to 15°C and left under vigorous stirring for 2 hours. The precipitate was filtered and washed with bistilled water (15 ml) dried in an oven at a temperature of 50°C. The purification yield was 79.1% and the HPLC purity was 99.2%.

### EXAMPLE 4

### 6α,9α,Difluoro-11β-dihydroxy-16α-methyl-3-oxo-17α(propionyloxy)androsta-1,4-diene-17-α(propionyloxy)androsta-1,4-diene-17β-carbothioic acid S-(fluoromethyl)ester (fluticasone propionate).

A suspension of 6α-9α-difluoro-11β-hydroxy-16-α-methyl-3-oxo-17 -α-propionyloxy)androsta-1,4diene-17β-carbothioic acid (6.1 g, 13.5 mmol) in dimethylformamide (65ml) was treated with potassium carbonate (11.5g, 108.5 mmol) for 30 minutes, cooled to 0-5°C and left to react until obtaining complete salification. The mixture was then slowly added with a solution of bromofluoromethane (53.4 g, 472 mmol) in dimethylformamide (157 ml) and left to react at room temperature until complete conversion of the starting product. The mixture was then precipitated in a HCl 2N solution (600 ml) and cooled to 0-5°C for at least 8 hours, filtered, the precipitated was then washed abundantly with water and dried in an oven at 60°C. 10 g of a raw product were obtained having a titre in fluticasone propionate equal to 99.2%. This product was further purified by crystallisation under reflux in tetrahydrofurane/dichloromethane in volume ratio 5:1 and successive evaporation of dichloromethane. This crystallisation allows to obtain fluticasone propionate with a titre of 99.5% with a reaction yield equal to 83%, optionally another crystallisation under reflux may be carried out in ethylacetate/butanol in volume ratio respectively of 5:11 with a reaction yield of 81% and titre in fluticasone propionate equal to 99.7%.
IR(KBr, cm⁻¹):3322(-OH); 2976-2880 (-CH-CH2), 1744 (OC(O));1661 (-C(O)),1614 (-C=C); 1453 (-ch);1071 (-CF).
¹H-NMR (CDCl₃,200Mhz,ppm),0.90 (d, 3H);1.00 (t,3H), 1.08 (S, 3H), 1.50 (s,3H), 2.38 (q, 2H), 5.60(m,1H); 5.97 (d, 2H);6.12 (s,1H); 6.30 (dd,1H); 7.25 (d, 1H).
¹⁹F-NMR-89.78, 111.83 (6-α,9α-difluoro); 117.54 (25-fluoro)

### COMPARATIVE EXAMPLE 4A

A suspension of 6α-9α-difluoro-11β-hydroxy-16-α-methyl-3-oxo-17 -α-propionyloxy)and rosta-1,4diene-17β-carbothioic acid (5.0g,11.0mmol) in dimethylformamide (65 ml) was treated with potassium carbonate (9.3 g , 88.0 mmol) for 30 minutes and left to react at 0-5°C until obtaining complete salification. The reaction mixture was then slowly added with a solution of chlofluoromethane (26.4 g, 385 mmol) in dimethylformamide (157 ml) and left to react at room temperature until achieving a complete conversion. The mixture was then precipitated in an HCl 2N solution (500 ml), cooled to 0-5°C and filtered. The precipitate thus obtained was purified by crystallisation under reflux in the solvent mixture tetrahydrofurane (10 vol) /dichloromethane (2 vol), successive remotion of dichloromethane and cooling to 0-5°C. The procedure allows to obtain a product with a purity degree of 96.5% with a yield of 80%. The successive purification carried out under reflux in the solvent mixture:ethylacetate(5 vol)/ butanol (1 vol) allows to obtain the final product with a purity degree of 83% and a HPLC purity of 98.3%.

## Claims

1. A process for preparing 17-β-carboxylic acid of general formula (I) 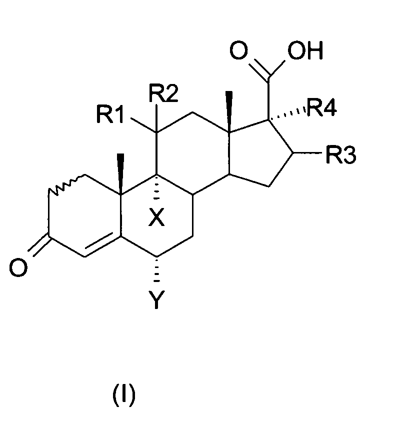 Wherein the symbol: 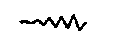
indicates the presence of a single or a double bond, R1 and R2 respectively represent a hydroxy group and a hydrogen atom or they form together an oxo group,
X and Y equal or different from each other are Cl, F, R3 respectively represents the 16α or 16β methyl, R4 is a hydroxy or a C1-C6 alkanoyloxy group,
comprising the oxidation reaction at the 21 carbon atom of the compound of general formula (II) 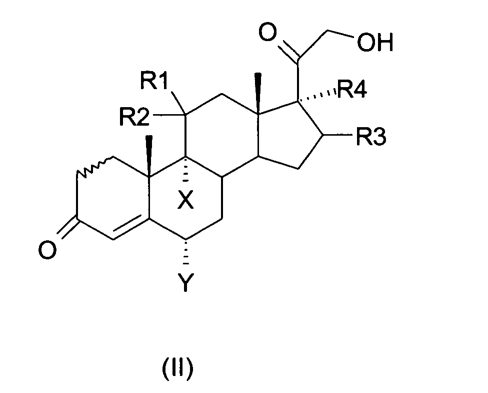 wherein R1, R2 X, Y, R3 and R4 and the symbol 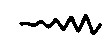
have aforementioned meanings, **characterised in that** said oxidation is carried out by using hydrogen peroxide as the oxidising agent.

2. The process according to claim 1 wherein the molar ratio of hydrogen peroxide/reactant of formula (II) is comprised between 5:1 and 50:1

3. The process according to claim 2 wherein said ratio is comprised between 20:1 and 10:1.

4. The process according to anyone of claims 1-3, wherein said oxidation reaction is carried out in the presence of a base and of a polar solvent, said solvent being optionally in admixture with water.

5. The process according to claim 4, wherein said base is selected from the class consisting of: alkaline metal carbonate or hydrogen carbonate salts, alkaline metal phosphate or hydrogen phosphate salts, mono and dialkylamines, heterocyclic amines, bicyclic amines.

6. The process according anyone of claims 4 and 5, **characterised in that** said polar solvent is selected from the class consisting of an aprotic dipolar solvent, protic polar solvents, aprotic polar solvent.

7. The process according to anyone of claims 4-6 as the reactant of formula (II) is flumethasone.

8. The process according to claim 7, wherein the oxidation process is carried out in the presence of potassium carbonate in a mixture of dimethylformamide and methanol at a temperature of 10°C.

9. The process according to anyone of claims 1-8, **characterised in that** it is used for the preparation of and fluticasone and esters thereof.

10. The process according to claim 9, **characterised in that** it is used for the preparation of fluticasone propionate.

11. The process according to claim 10, wherein the 17-β-carboxylic acid of formula (I) wherein X=Y=F, R1=β-OH, R2=α-H, R4 =OH or propionyloxy, R3 = α-methyl and the symbol: 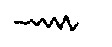
indicates a double bond.

12. The process according to anyone of claims 10 and 11, comprising before carrying out the oxidation reaction the following steps:
a) reacting the compound of formula (II) wherein X=Y=F, R1=β-OH, R4 =OH R3 = α-methyl and the symbol: 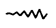
indicates a double bond,
with a trialkyl orthopropionate of formula (III): 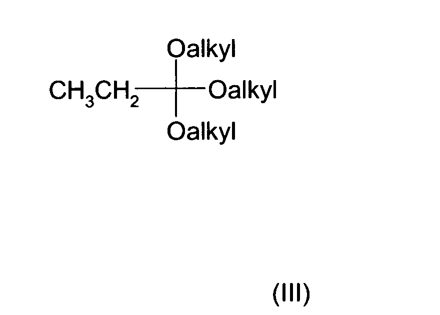
wherein "alkyl"means a C1-C5 linear or branched alkyl residue, in an aprotic polar solvent thereby obtaining the compound of formula (IV) wherein "alkyl" has the aforementioned meanings: 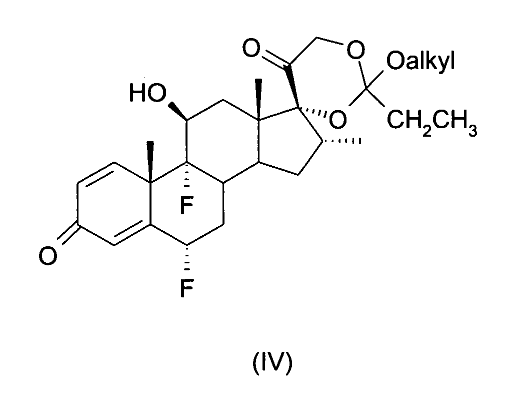 hydrolysing the cyclic adduct of formula (IV), in the presence of an organic carboxylic acid in an alcoholic solvent optionally in the presence of an apolar solvent, thereby obtaining the compound of formula (II) wherein R4=OCOCH2CH3, whereas X,Y, R1, R2 and R3 and the symbol 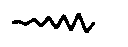
have the aforementioned meanings.

13. The process according to claim 12, wherein "alkyl" in the trialkyl orthoformiate of formula (III) utilised in step (a) is selected from ethyl, propyl, butyl.

14. The process according to claim 13, wherein "alkyl" is ethyl.

15. The process according to anyone of claims 12-14, wherein said aprotic polar solvent used in step (a) is selected from the group consisting of tetrahydrofurane, dioxane, ethyl acetate.

16. The process according to claim 15, wherein said solvent is ethyl acetate.

17. The process according to anyone of claims 12-16, wherein step (a) is carried out at a temperature ranging from 15°C to reflux temperature.

18. The process according to claim 17, wherein said temperature is room temperature.

19. The process according to any one of claims 12-18, wherein in step (b) said organic carboxylic acid is acetic acid.

20. The process according to anyone of claims 12-19, wherein in step b) the alcoholic solvent is selected from the group consisting of methanol, ethanol, n-propanol, isopropanol, butanol or mixtures thereof optionally in the presence of the apolar solvent selected from the group consisting of: dichloromethane and chloroform.

21. The process according to claim 20, wherein said apolar solvent is dichloromethane.

22. The process according to anyone of claims 12-21, wherein step (b) is carried out at a temperature ranging from 20 to 60°C.

23. The process according to claim 22, wherein said temperature is 42°C.

24. A process for preparing fluticasone propionate comprising reacting the 17-β carbothioic acid of formula (VI) 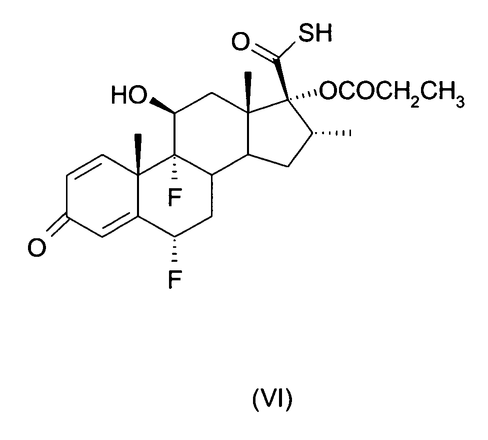 with bromofluoromethane.

25. The process according to claim 24 wherein bromofluoromethane dissolved in a diprotic polar solvent is added at portions to a suspension of the carbothioic acid (VI) in the same solvent.

26. The process according to claim 25 wherein said diprotic polar solvent is a C2-C5 alkylamide of a carboxylic acid.

27. The process according to claim 26 wherein said C2-C5 alkylamide of a carboxylic acid is selected from the group consisting of dimethylacetamide and dimethyl formamide.

28. The process according to anyone of claims 24-27 wherein the reaction temperature is comprised between -20 and + 30°C.

29. The process according to anyone of claims 24-28 for preparing crude fluticasone propionate having a purity degree of 99.2%.

30. The process according to anyone of claims 24-29, further comprising at least one crystallisation step.

31. The process according to claim 30, comprising two crystallisation steps.

32. The process according to claim 31, wherein the first crystallisation step is conducted with a process comprising :
i) dissolving the raw product under reflux in tetrahydrofurane/dichloromethane in volume ratio 5:1
ii) evaporating dichloromethane, favouring the precipitation of the purified product,
iii) filtering and drying the purified product.

33. The process according to anyone of claims 31-32 wherein the second crystallisation step is carried out with a process comprising
i) dissolving the raw product under reflux in ethylacetate/butanol in volume ratio 5:11
ii) cooling the solution obtained to precipitate the purified product
iii) filtering and drying the purified product.

34. The process according to anyone of claims 30-31 allowing to obtain a fluticasone propionate having a purity degree higher than 99.5%.

35. Fluticasone propionate having a purity degree higher than 99.5%.

36. Fluticasone propionate according to claim 34 having a purity degree of 99.7%.
